# EUROPEAN PATENT SPECIFICATION

(11) **EP 2 863 893 B1**
(45) Date of publication and mention of the grant of the patent: **27.08.2025**
(21) Application number: 13807160.0
(22) Date of filing: 21.06.2013
(51) Int. Cl.: A61M 37/00, A61K 9/16, A61K 47/02, A61K 9/00

(54) **METHOD AND COMPOSITION FOR DELIVERING A COMPOUND THROUGH A BIOLOGICAL BARRIER**
VERFAHREN UND ZUSAMMENSETZUNG ZUR FREISETZUNG EINER VERBINDUNG DURCH EINE BIOLOGISCHE BARRIERE
PROCÉDÉ ET COMPOSITION D'ADMINISTRATION D'UN COMPOSÉ À TRAVERS UNE BARRIÈRE BIOLOGIQUE

(30) Priority: 22.06.2012 AU 2012902651; 26.04.2013 AU 2013901464
(43) Date of publication of application: 29.04.2015
(73) Proprietor: UniSA Ventures Pty Ltd, South Australia 5059 (AU)
(72) Inventor: PROW, Tarl W, Sunnybank Queensland 4109 (AU); SOYER, H. Peter, Holland Park Queensland 4121 (AU)
(74) Representative: Witthoff Jaekel Steinecke Patentanwälte PartG mbB
(86) International application number: PCT/AU2013/000670
(87) International publication number: WO 2013/188926

(56) References cited:
- WO-A1-01/93947
- WO-A1-97/04832
- WO-A2-00/72827
- KENDALL M A F ET AL: "Transdermal ballistic delivery of micro-particles: investigation into skin penetration", ENGINEERING IN MEDICINE AND BIOLOGY SOCIETY, 2000. PROCEEDINGS OF THE 22ND ANNUAL INTERNATIONAL CONFERENCE OF THE IEEE 23-28 JULY 2000, PISCATAWAY, NJ, USA,IEEE, vol. 3, 23 July 2000 (2000-07-23), pages 1621 - 1624, XP010530801, ISBN: 978-0-7803-6465-3
- TARL W PROW ET AL: "Nanoparticles and microparticles for skin drug delivery", ADVANCED DRUG DELIVERY REVIEWS, ELSEVIER, AMSTERDAM, NL, vol. 63, no. 6, 31 January 2011 (2011-01-31), pages 470 - 491, XP028374105, ISSN: 0169-409X, [retrieved on 20110209], DOI: 10.1016/J.ADDR.2011.01.012
- KIS, E.E. ET AL.: "Devices for intradermal vaccination", VACCINE, vol. 30, 18 November 2011 (2011-11-18), pages 523 - 538, XP028348164
- CHAMPION, J. ET AL.: "Making polymeric micro- and nanoparticles of complex shapes", PNAS, vol. 104, no. 29, 2007, pages 11901 - 11904, XP055164687
- BOONEN J ET AL.: "Skin penetration of silica microparticles.", DIE PHARMAZIE, vol. 66, no. 6, 2011, pages 463 - 464, XP055164689
- TINKLE, S. ET AL.: "Skin as a Route of Exposure and Sensitization in Chronic Beryllium Disease", ENVIRON. HEALTH PERSPECT., vol. 111, 2003, pages 1202 - 1208, XP055164691
- PROW, T. ET AL.: "Nanoparticles and microparticles for skin drug delivery", ADVANCED DRUG DELIVERY REVIEWS, vol. 63, 2011, pages 470 - 491, XP028374105

## Description

### TECHNICAL FIELD

This disclosure relates to a method and a composition of delivering a compound through a biological barrier.

### BACKGROUND ART

The oral administration of many drugs and other bioactive compounds is problematic due to the risk of degradation of the compounds in the gastrointestinal tract and/or elimination by the liver. Moreover, some drugs cannot effectively diffuse across the intestinal mucosa. Patient compliance may also be a problem, for example, in therapies requiring that pills be taken at particular intervals over a prolonged time.

Significant research has been conducted in recent years into the transport of drugs and therapeutic agents across biological barriers in the body, e.g., the skin, mucosal membranes, such as the oral mucosa or vaginal/cervical epithelium, the blood-brain barrier etc.

In the case where the biological barrier is skin, a major obstacle that must be overcome in developing effective transdermal delivery systems is the naturally low permeability of skin. Skin is a structurally complex, relatively thick membrane that provides an effective barrier to the entry of substances into the body. In human skin, the outer layer, the stratum corneum (SC), is approximately 10 to 20 µm thick and consists of a stack of 15 to 25 flattened, cornified cells embedded in a matrix of intercellular lipid. One function of this layer is to form a protective barrier, preventing the entry of hazardous environmental material and microorganisms into the body. Consequently, this layer is also the main barrier that must be overcome to deliver drugs or other therapeutic substances through the skin. Once across the SC, substances are then able to cross the viable epidermis and diffuse into the papillary dermis where they can enter the capillaries and be absorbed into the systemic circulation, enter lymphatic vessels or diffuse into the dermis and underlying tissue compartments.

Some bioactive compounds may be absorbed through the SC by topical application, e.g. by manually rubbing a preparation containing the compound into the skin. However, this technique is inefficient and is limited to absorption of relatively small bioactive compounds.

One common technique for delivering drugs across a biological barrier such as skin is the use of a hypodermic needle, such as those used with standard syringes or catheters. The use of such needles generally causes pain; and may cause local damage to the skin at the site of insertion; bleeding, which increases the risk of disease transmission; and a wound sufficiently large to be a site of infection among many other disadvantages. Needle techniques also generally require administration by one trained in the use of needles. The needle technique also may be undesirable for long term, controlled continuous drug delivery.

Recent advances in transdermal delivery devices have included transdermal patches, which rely on diffusion of small molecules across the SC, and microneedle arrays, which pierce the SC to facilitate delivery of compounds. However, transdermal patches are not effective for delivering relatively large molecules which are not able to diffuse across the SC. Moreover, microneedle arrays have the disadvantages of requiring supporting structures and/or applicators, which add to the complexity and cost of manufacture and use of such devices. Another disadvantage of transdermal patches and microneedle arrays is that their size necessarily limits the surface area for delivery of the bioactive compounds. Microneedle arrays limit drug application to the area and shape of the array.

There is accordingly a need for a means for delivering drugs and other bioactive compounds transdermally which overcomes or at least alleviates one or more of the disadvantages of the prior art.

### SUMMARY OF THE DISCLOSURE

In one aspect there is disclosed a method for delivering a compound through a biological barrier including:
providing a composition including a plurality of elongate microparticles and the compound, and
applying a force between 0.01 Newtons and 10 Newtons by rubbing or massaging the composition on a surface of the barrier manually or by using an applicator so that at least some of the microparticles penetrate the biological barrier and thereby facilitate delivery of the compound through the biological barrier, wherein the microparticles have a geometry and strength sufficient to penetrate the biological barrier; comprise silica, have an average length of from 90 µm to 510 µm; and have a width or diameter of 520 µm or less.

The compound may be a bioactive compound.

By using microparticles as a means of penetrating the biological barrier, as opposed to fixed array microneedles, the need for a solid support or fixed substrate, and large impact/velocity driven applicators, can be minimized or obviated. Accordingly, the microparticles are independently moveable and are not attached to a solid support or a fixed substrate. The cost and complexity of manufacturing the microparticles of this disclosure are therefore likely to be significantly less than those for microneedle arrays. Moreover the delivery of compounds using the microparticles is more effective than transdermal patches, particularly for relatively large molecules, by virtue of the penetration of the biological barrier by the microparticles and attendant increase in permeability. In addition, in comparison with transdermal patches and microneedle arrays, the microparticles can deliver compounds over a large and irregular shaped area, and to inaccessible surfaces (such as vaginal tracts) due to the absence of the solid support and a fixed size and shape array. As mentioned above, the step of applying a force is carried out manually by rubbing and/or massaging either by hand or by using an applicator.

Without wishing to be limited by theory, it is believed that the penetration of the biological barrier by the microparticles creates pathways for delivery of the compounds across the biological barrier. The compounds may be delivered through the pathways simultaneously with and/or subsequent to their creation. For example, the compound may be pushed through the biological barrier by the microparticles as they create the pathways, and/or they may pass through the pathways subsequent to their creation, for example by being rubbed or massaged into a previously formed pathway.

In an embodiment of the methods disclosed, the biological barrier may be the stratum corneum of human skin. In another embodiment, the biological barrier may be the stratum corneum of animal skin.

As mentioned above, the force applied to the microparticles is greater than 0.01 Newtons and the maximum force is 10 Newtons. In one embodiment, the applied force may be between 0.1 Newtons and 10 Newtons, such as between 0.2 Newtons and 10 Newtons.

The inventors have found that low angle microparticle penetration results in maximal disruption of and delivery across the biological barrier (e.g. skin) as compared to application using perpendicular fixed substrate microneedle arrays.

The microparticles may be orientated at an acute angle with respect to the skin surface to facilitate penetration of the biological barrier.

In an embodiment, the microparticles may be orientated, such as to penetrate the skin at an angle of less than 45 degrees, such as between approximately 5 to 30 degrees, for example between 7 and 25 degrees.

In a further aspect there is disclosed a composition for use in delivering a compound through a biological barrier, wherein the composition includes a plurality of elongate microparticles having a geometry and strength sufficient to penetrate the biological barrier upon application of a force of between 0.01 Newtons and 10 Newtons by rubbing or massaging manually or by using an applicator, comprising silica, having an average length of from 90 µm to 510 µm, and having a width or diameter of 20 µm or less; and wherein the composition includes the bioactive compound to be delivered, wherein the bioactive compound forms a coating on the elongate biocompatible silica microparticles; or the composition comprises a gel, emulsion, cream, ointment or solution.

The microparticles are preferably biocompatible. By "biocompatible" is meant that the microparticles do not cause a toxic, injurious, or adverse immunological response in living tissue.

Depending on the composition of the microparticles, the microparticles that penetrate the skin barrier may remain therein for 3-4 weeks and be removed by natural turnover of skin (such as where the microparticles are formed from silica).

In an embodiment, the microparticles comprise a material having sufficient strength and rigidity to be able to penetrate the biological barrier under an applied force of at least 13MPa, wherein the material comprises silica. Silica is conveniently used due to its relative low cost, suitable mechanical properties, biocompatibility and availability.

In an embodiment the elongate microparticles are dimensioned to penetrate through a stratum corneum of skin. The dimensions are indicated further below.

The elongate microparticles may have a high aspect ratio. The aspect ratio (length:width) may be at least 10:1, such as at least 15:1. In some embodiments the aspect ratio may be greater than 20:1. The upper limit on aspect ratio may be around 200:1, such as about 150:1. In some embodiments, the maximum aspect ratio is 100:1.

The maximum length of the elongate microparticles may be 800 µm and is preferably 500 µm. The average length of the elongate microparticle varies from about 90 to about 510 µm.

The maximum width or diameter of the elongate microparticles is less than 20 µm. For some materials, the width may be less than 12µm, such as between 5 and 10 µm, in the case of silica.

In an embodiment, the microparticles are substantially uniform in size. At least 50%, and preferably at least 70% of the microparticles, may have lengths within 80% of the median length. In an embodiment, at least 80% of the microparticles have lengths within 80% of the median length.

In an embodiment the elongate microparticles may be hollow, solid or a combination thereof.

In an embodiment at least some of the elongate microparticles have one or more substantially flat ends.

In an embodiment at least some of the elongate microparticles ("EMPs") have a tapered end geometry. The EMPs preferably have an end angled at less than 180° and may be less than 20° such as less than 10°.

In an embodiment at least some of the elongate microparticles have one or more substantially convex ends.

In an embodiment the end surface area of the elongate microparticles may be a maximum of about 2000 square micrometers.

In an embodiment the composition further includes a bioactive compound.

In an embodiment the bioactive compound may comprise one or more of a therapeutic compound, a cosmeceutical, a pharmaceutical, a nutraceutical, a diagnostic agent, a vaccine, siRNA or an anti-oxidant.

Thus, the composition is an admixture of the microparticles and the bioactive compound. The admixture may be a dry or semi-dry preparation, such as a EMP containing powder or paste.

In an embodiment the composition may contain the microparticles in a concentration of 1 mg/mL or higher, such as at least 10 mg/mL, preferably at least 100 mg/mL. In an embodiment, the composition may contain microparticles in a concentration of up to 1 g/mL.

In an embodiment the bioactive compound may form a coating on the microparticles.

In an embodiment the composition may further include a carrier, such as a saline fluid, alcohol, hydrogel or a cream.

In an embodiment the composition may further include a lubricant.

In an embodiment, the composition comprises a mixture of the microparticles, and a pre-existing formulation, such as a gel, emulsion, cream, ointment or solution.

In an embodiment the microparticles may be soluble in vivo.

### BRIEF DESCRIPTION OF THE DRAWINGS

Notwithstanding any other forms which may fall within the scope of the methods, composition and kit as set forth in the Summary, specific embodiments will now be described, by way of example only, with reference to the accompanying drawings. It is to be understood that only the microparticles as characterized in the claims are part of the invention.
Figure 1 is a photomicrograph of microparticles.
Figures 2 to 4 illustrate particle size distributions of "small", "medium" and "long" microparticles, respectively.
Figures 5 and 6 illustrate penetration depth of sodium fluorescein in previously frozen skin tested at 33°C with "small", "medium" and "long" microparticles.
Figures 7 and 8 illustrate the effect of application forces on penetration of sodium fluorescein using microparticles in freshly excised, non-diseased, human skin.
Figures 9a and 9b are scanning electron micrographs of 'short' and 'long' elongate microparticles (EMPs).
Figure 9c illustrates EMP mean length distribution of the 'short' and 'long' EMPs .
Figures 10a to 10c illustrate experimental results from low angled penetration of the EMPs applied to excised pig skin.
Figures 11a to 11g illustrate experimental results from low angled penetration of the EMPs applied to a volar forearm of volunteers.
Figures 12a to 12l compare delivery of sodium fluorescein to ex vivo pig skin using topical application (no EMP) (a, d, g and j), short EMPs (b, e, h and k) and long EMPs (c, f, i and l).
Figure 13 compares delivery of sodium fluorescein to in vivo pig skin using topical application (no EMP) (a, d, g and j), short EMPs (b, e, h and k) and long EMPs (c, f, i and I).
Figures 14a and 14b illustrate the effect of application force on EMPs for delivery of sodium fluoroscein.
Figures 15a to 15i illustrate a comparison of delivery profile of sodium fluoroscein by topical application (no EMP) (a, d and g), by using microneedles (b, e and h) and by using EMPs (c, f and i).
Figures 16a to 16d illustrate enhanced delivery of sodium fluorescein (a and b), Vitamin A (c), and Vitamin B₃ (d) to pig skin, by topical application (no EMP), short EMPs and long EMPs.
Figures 17a to 17e illustrate the *ex vivo* delivery of Vitamin E, Vitamin B₃ and ¹⁴C-aminolevulinic acid to human skin using a third embodiment of the EMPs.
Figures 18a to 18f illustrate the *in vivo* delivery profile of sodium fluorescein integrated density within skin of volunteers over a two week period. Light grey represents EMP enhanced delivery and black represents no EMPs.
Figure 19 illustrates the enhanced immune response when a DNA vaccine to West Nile virus is delivered to mice by long EMPs compared to intramuscular administration.
Figure 20 shows penetration and delivery of EMPs having a diameter of (a) 12 µm , (b) 20 µm and (c) 39 µm, respectively. Figure 20(d) shows the number of EMPs penetrated per mm² vs diameter. Figure 20 (e) shows integrated density vs depth from skin surface. Figure 20(f) shows NaF intensity versus EMP diameter.
Figure 21(a) and (b) are microscopy images of the two different end geometries of a silicon EMP, namely an included end face angle of (a) 180° and (b) 6°, respectively.

### DETAILED DESCRIPTION OF SPECIFIC EMBODIMENTS

In a preferred embodiment, the microparticles herein are used for delivering bioactive compounds through a biological barrier in the form of the stratum corneum of human skin. However, it is to be appreciated that the microparticles may also be used for animal health applications, such as in the delivery of vaccinations and treatment of various skin diseases in farm stock.

The composition of the preferred embodiment comprises an admixture of microparticles and a bioactive compound, wherein themicroparticles comprise silica and are in the form of substantially cylindrical microfibers having a length in the range of 50µm to 400µm and a width of approximately 8µm.

The bioactive compound is one or more of a cosmeceutical, a pharmaceutical, or a therapeutic compound.

The composition is applied to the stratum corneum by manually applying a force in the order of 0.4N, such as by massaging the composition into the SC by hand or with an applicator. The silica microparticles are freely movable in relation to each other under action of the applied force. At least some of the microparticles penetrate the SC and, it is believed, create pathways for delivery of the bioactive compound/s to the viable epidermis either simultaneously with and/or subsequent to the pathway creation.

### EXAMPLES

In the Examples that follow, the results of a number of experiments are presented that were performed using sodium fluorescein dye (and other compounds as described in Example 3) as a model compound in both *in vitro* and *in vivo* models of human and pig skin. Sodium fluorescein (NaF) is a gateway model drug for volunteer studies. Both *ex vivo* and *in vivo* tissue samples were analysed *en face* using fluorescence (excited at 488 nm) and reflectance (excited at 750 nm) confocal microscopy (Vivascope^{®} 1500 Multilaser, Lucid Inc., USA). The microscope also consisted of a dermatoscope, which was used to image and position the skin prior to microscopy. The samples were optically sectioned at 1.5 µm intervals from the surface of the skin down to a depth of 150 µm. Each optical slice consisted of a focal area of 500 x 500 µm².

The *en face* image stacks were analysed using Image J Software (NIH, USA). The reflectance confocal microscopy image stacks were used to characterize the number of EMPs that penetrated the skin, the length of penetrated microparticles, their penetration depth and EMP penetration angle. The EMPs were distinguished by low intensity bordering a high intensity region resulting in their unique rectangular appearance compared to the surrounding tissue. The microparticle penetration depth and length were converted to Cartesian coordinates and the data used to construct an accurate representation of the EMPs within the skin using both Image J and Autodesk Inventor Professional 2012.

In relation to the sodium fluorescein signal, an area 300 x 300 µm² in size was selected in the center of the image and the raw integrated density was measured throughout the entire stack. The data were then divided by the analysed area (9000 µm²) resulting in the sodium fluorescein integrated density per µm².

### Example 1

### Transdermal and transmucosal delivery of sodium fluorescein dye using EMPs of varying length and application force.

EMPs were fabricated using two different methods (one technique for the 'short' and another for the 'medium' and 'long'). The first method used high energy ball milling to micronize 20 mg of glass wool. The samples were placed in a 2 ml lysing matrix tubes with 40 mg of 1.4 mm ceramic beads (MP Biomedicals, USA) and milled for 45 seconds (Bio 101 FastPrep FP120-120V, Thermo Savant, USA). A sample of the EMPs was assessed using stereomicroscopy and the milling process repeated if necessarily to achieve the desired size range (< 50 µm). The process was repeated until the desired amount of 'short' EMPs was produced.

The medium and longer length EMPs were fabricated using an in-house micro-chopping technique. Glass wool was spread evenly over a chopping board forming an approximate 1 mm layer. A stainless steel circular punch, 350 µm ('medium') or 750 µm ('long') in diameter was then used to chop the wool into microparticles. A sample of the EMPs was assessed using stereomicroscopy to determine the size distribution.

Elongate silica microparticles (EMPs) in the form of cylindrical silica microfibers having a length in the range of 50µm to 800µm and a width of 8µm were shown to greatly enhance transdermal delivery of a small hydrophilic molecule (sodium fluorescein dye).

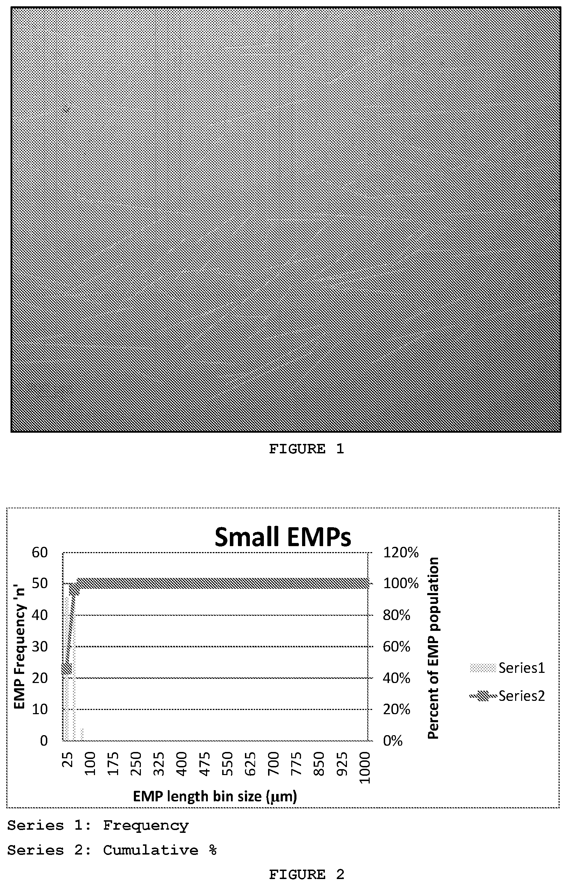

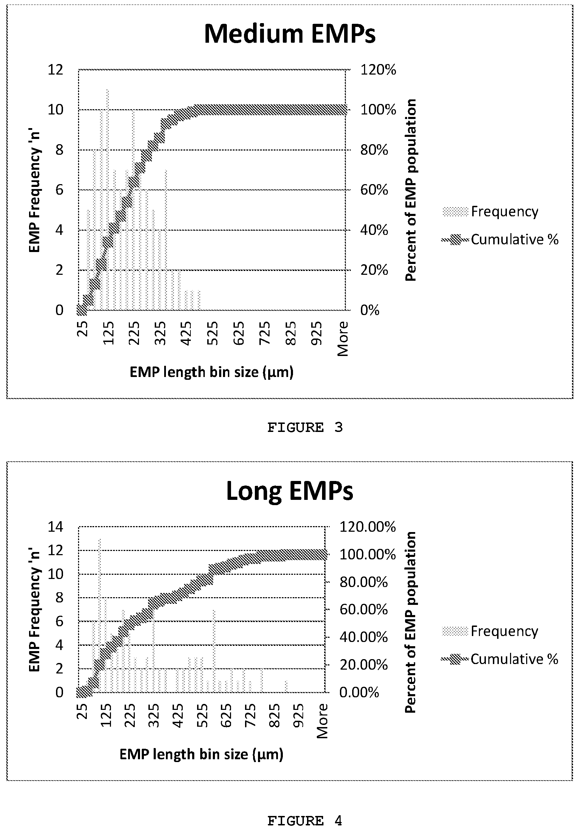

Figure 1 is a photomicrograph of the EMPs. The EMPs were classified according to length as "small", "medium" and "long". Figures 2, 3 and 4 illustrate the distribution of particle size for small, medium and long EMPs, respectively. Each figure illustrates the numbers of EMPs (bars, left vertical axis) having a particular length (in microns, horizontal axis) as well as the cumulative percent of all EMPs, shown by the squares.

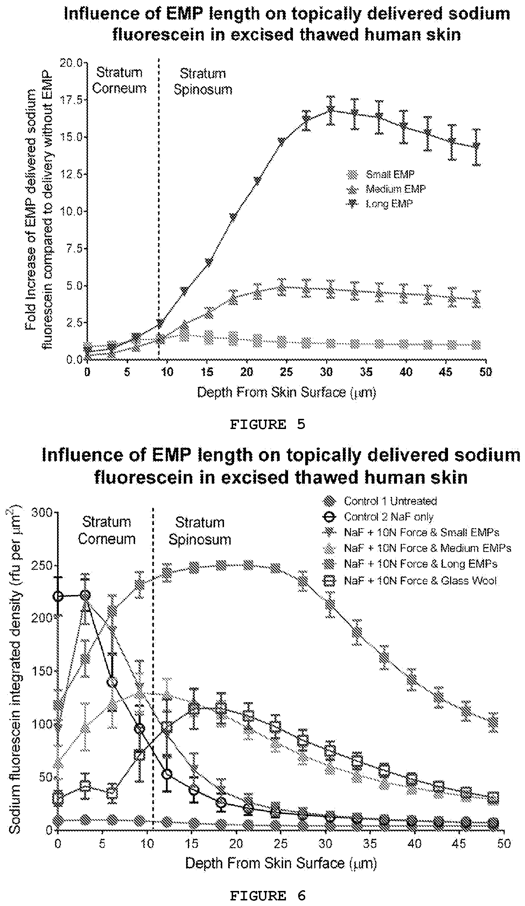

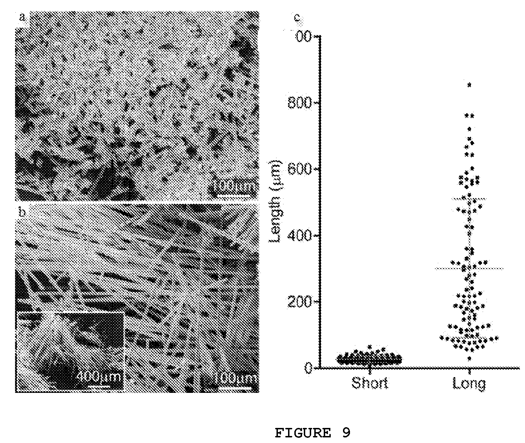

A mixture was formed of by taking 50µl of sodium fluorescein dye (1mg/mL) and mixed with or without 5mg of silica EMP This mixture was applied to previously frozen or freshly excised human skin under varying conditions of EMP length and/or application force. The results are illustrated in Figures 5 to 8. The silica EMPs had a range of end geometries (flat, angled and irregular) arising from the manufacturing method which involved the fracturing of glass wool (this can be seen, for example, in Figure 9, discussed below).

Figure 5 shows the depth of penetration of sodium fluorescein (NaF) in thawed human skin when applied in a mixture with small (squares), medium (upright triangles) or long (inverted triangles) EMPs when viewed with fluorescence confocal microscopy at a point 15 minutes following the application of dye. Excised human skin (non-diseased) was obtained from female, Caucasian, abdominal plastic surgery patients who gave their informed consent to participate in the study. The skin was stored at -20°C and then brought to room temperature prior to use. The results indicate that the greatest amount of sodium fluorescein penetrated into the stratum spinosum when long EMP were used. In Fig., 5 the signal to noise ratio measures the fold-increase of sodium fluorescein delivered with EMPs compared to sodium fluorescein delivered without EMPs. It is measured by dividing the sodium fluorescein pixel intensity achieved with EMPs by the sodium fluorescein pixel intensity achieved without EMPs. The pixel intensity is measured using Image J (National Institute of Health, USA) imaging software, where the software quantifies the brightness of intensity on a scale of 0 (nothing) to 255 (maximum intensity).

Similar results are seen in Figure 6, in which the greatest amount of sodium fluorescein penetrated using long EMPs (upper squares). In Fig. 6 the 'Integrated density' refers to the integrated density of sodium fluorescein pixel intensity (rfu = relative fluorescent units).

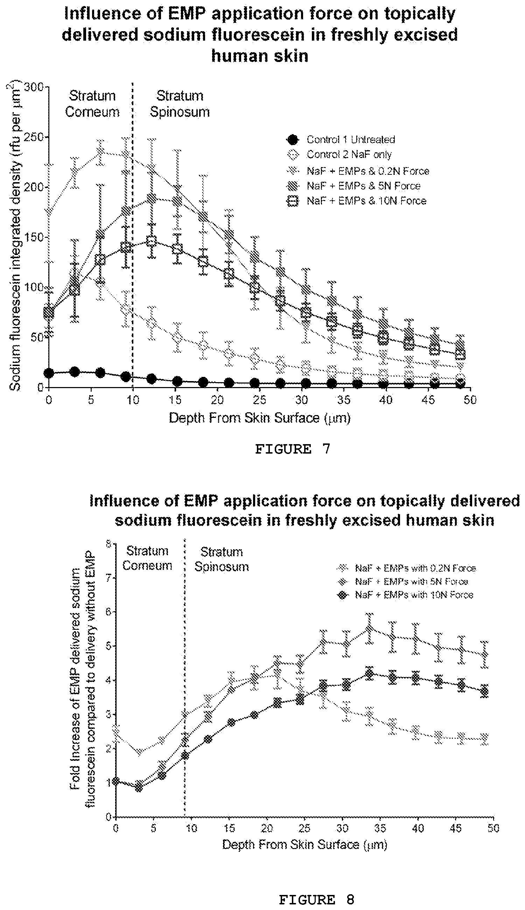

Figures 7 and 8 illustrate the effect of application forces on the penetration of sodium fluorescein in conjunction with 'long' EMPs. Measurements were made 15 minutes after application to freshly excised, non-diseased human skin (from female, Caucasian, abdominal plastic surgery patients). Comparison of the penetration of sodium fluorescein without EMPs (lower squares) with penetration when EMPs are present indicates that the EMPs facilitate penetration. Application forces of 0.2N, 5N and 10N were all applied using a gloved finger on a scale. The excised skin is place on the scale and the finger is applied to the EMPs/skin until a weight is achieved on the scale corresponding to either 0.2, 5 or 10 N.) All forces are effective in improving penetration of sodium fluorescein into the stratum spinosum. There was an apparent optimum application force of 5N under the conditions of the tests. However, at higher penetration depths, there was no significant difference in results between 5N and 10N. The pathways that were created for the sodium fluorescein dye molecules by massaging the silica EMPs into the skin resulted in a several-fold increase in the delivery of sodium fluorescein dye across the stratum corneum as compared with simple diffusion of sodium fluorescein using topical application (without EMP).

In a separate experiment it was demonstrated that medium length EMP of the disclosure can also boost the penetration of EMP through mucosal skin surfaces. EMP used in this experiment varied in length (mean, 301.0±209.5µm) such that 50% of the microparticle population had a length between 118.0 and 477 µm. In these in vivo mouse experiments, a 10µl quantity of sodium fluorescein (1mg/ml) was pipetted into the vaginal tracts of mice. In one group of mice, dye application occurred only after prior application of a small quantity of EMP (5mg). The dye solution was gently massaged with a rod-like applicator. Vaginal tracts were removed at sacrifice and mounted for cryosectioning. When sodium fluorescein was applied to the mucosa in the absence of EMP, minimal fluorescence was observed within the tissue sections. However, when sodium fluorescein was administered in conjunction with EMP there was a significantly greater fluorescence signal detected throughout the surface of the epithelium. Analysis of the signal intensity demonstrated that the EMP assisted sodium fluorescein to penetrate within the epithelim to about 60µm. This is more than 4 times deeper within the epithelium compared to delivery in the absence of EMP. This suggests that EMP can enhance the delivery of agents across both mucous containing and non-mucous containing skin or membrane and are useful for facilitating delivery to inaccessible surfaces (e.g. vaginal tract).

### Example 2

### Angle of penetration of EMPs and Comparison of EMPs with Fixed Substrate Microneedles.

Example 2 demonstrates that low angle EMP penetration enhances the delivery profile of bioactive compounds.

Example 2 is an exemplary embodiment of EMPs to illustrate that low angle EMP penetration at least results in maximal disruption and delivery within the skin compared to perpendicular fixed substrate microneedle application.

Figures 9a and 9b are micrographs of EMPs obtained by Scanning Electron Microscopy. The EMPs presented in this example had a diameter of 9.3 ± 0.9 µm (mean±SD) (Figure 9a and b). Two different populations were fabricated that resulted in either a 'short' EMP distribution as illustrated in Figure 9a or 'long' EMP distribution as shown in Figure 9b. Figure 9c illustrates mean length distribution of "short" EMPs and "long" EMPs. The 'short' EMPs had an approximate mean length of 27.5 ± 9.8 µm with 50% of the population being between 20.9 µm and 32.6 µm in length (Figure 9c). The 'long' EMPs had an approximate mean length of 301.0 ± 209.5 µm, with 50% of the population being between 118.0 µm and 477.7 µm in length (Figure 9c).

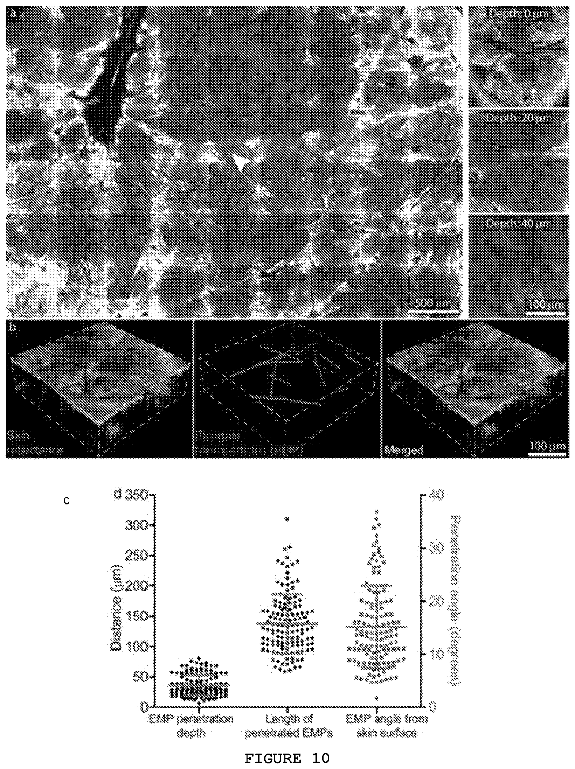

Figures 10a and 10b illustrate images of pig skin samples with EMP penetration using the 'long' EMPs as illustrated in Figure 9b. Excised pig ears sourced from an abattoir were washed with soap and water followed by rinsing. The ears were stored at -20°C until required. The ears were thawed and then gently shaved to remove excessive hair, then rinsed and pat dried. The ventral side of the ear was then separated from the cartilage using a scalpel. The excised skin was pinned down on a cork-board maintaining slight tension across the surface. The images in Figures 10a and 10b were obtained by using reflectance confocal microscopy. Only 'long' EMPs were applied to the skin as illustrated in Figure 10a and 10b, respectively. The white arrow in Figure 10a shows a single representative EMP. When 'short' EMPs were applied to the skin in a similar way they resulted in a negligible amount of EMPs penetrating into the skin and could not be analyzed. The long EMPs penetrated the skin in a relatively uniform manner within the treatment area, consisting of an average of 41 ± 11 EMPs per mm² as illustrated in Figure 10a. On puncturing the stratum corneum (approx. depth 0 -10 µm), the EMPs penetrated into the viable epidermis (approx. depth 20 - 40 µm) and dermo-epidermal junction / upper dermis (approx. depth 40 µm) as illustrated in Figures 10a (right panel) and 10b. The amount of EMPs varied with a larger number of EMPs detected within the epidermis (32 ± 12 EMPs per mm²) compared to the dermo-epidermal junction where 9 ± 6 EMPs were detected as illustrated in Figure 10a This is attributed to the angled shallow penetration of the EMPs, where the majority of the EMPs penetrate and disrupt the upper skin layers (0-40 µm in depth) compared to the deeper epidermal/dermal layers (40 µm and deeper)

Without wishing to be bound by theory, it is hypothesized that a lower number of EMPs within the dermo-epidermal junction can be attributed to the change in tissue composition from the cellular epidermis to the 'tougher' dermal collagen matrix.

Figure 10c illustrates distribution of EMP penetration depth, length of penetrated EMPs and EMP angle from skin surface. It appears that the EMP penetration depth may reflect that the dermal barrier is influencing penetration with the EMPs penetrating to a depth of 35.93 ± 17.15 µm (mean±SD) as illustrated in Figure 10c.

Even though EMP penetration depth was approximately 36 µm, the lengths of the EMPs within the skin were found to be 137.4 ± 48.79 µm (mean±SD) as also illustrated in Figure 10c. These results suggest a low angle of EMP penetration from the surface of the skin, which was determined to be 15.11 ± 7.71 degrees (mean±SD) as also illustrated in Figure 10c.

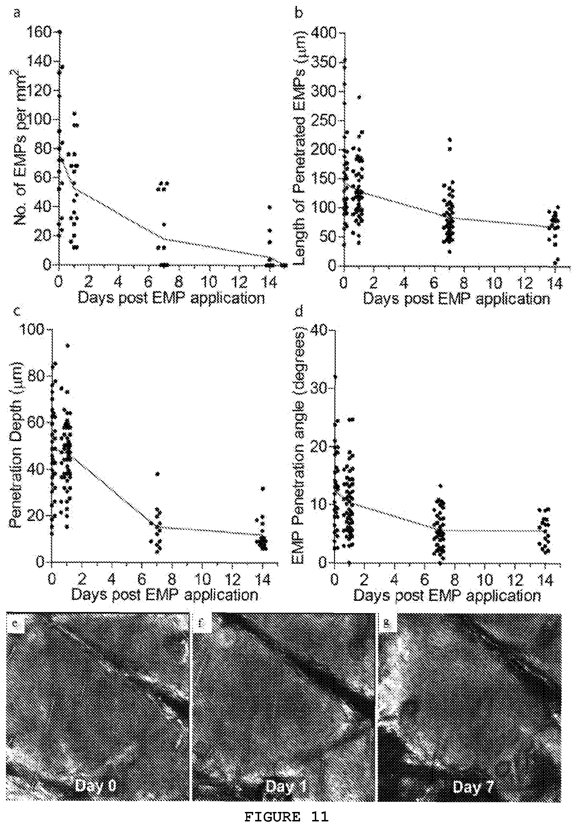

Figure 11 illustrates experimental results obtained from low angled penetration of EMPs applied to the volar forearm of four human, healthy volunteers using a plastic applicator as described in AU2012905650. Figure 11 shows the number, length, penetration depth and penetration angle of EMPs as measured using reflectance confocal microscopy. Figure 11a illustrates the variation in number of EMPs per mm² over a period of 14 days after low angled EMP application to the volar arm. Figures 11b and 11c illustrate length of penetrated EMPs and penetration depth of EMPs respectively over a period of 14 days after low angled EMP application. The number of EMPs decreased from 76 to 6 per mm² over a two week period with no EMPs detected by day 15 as illustrated in Figure 11a. Similar to what was observed in pig skin, the EMP penetration depth was less than the penetrated lengths of the EMPs, inferring angled penetration as illustrated in Figures 11b and 11c. Figure 11d illustrates penetration angle of EMPs over a period of 14 days after EMP application to the volar arm. This assumption was observed with angle of EMPs being 12.5 degrees as illustrated in Figure 11d. Figure 11e illustrates images of the volar arm obtained by reflectance confocal microscopy over a period of 7 days after low angled EMP application. The low angle of EMP penetration was visualized in the skin using reflectance confocal microscopy. Furthermore, the results in Figure 11 also show that over a time period of two weeks, the EMPs are naturally removed from the skin by transepidermal elimination.

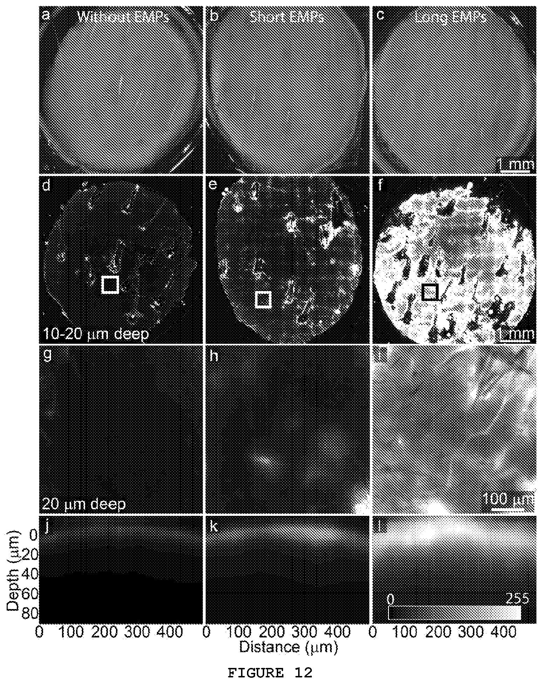

The previous Figures 9 to 11 illustrate that low angle of penetration results in enhanced skin disruption. A small hydrophilic molecule in the form of sodium fluorescein was used as a model drug and the EMP enhanced delivery profile resulting from low angle penetration of EMPS is illustrated in Figures 12 to 16.

Figure 12 represents comparison of delivery of sodium fluorescein to *ex vivo* pig skin. Pig skin was sourced and prepared for drug treatment as described for Figure 10. For EMP delivery, 50 µL of the payload solution (1 mg/mL sodium fluorescein) was gently mixed with or without 5 mg of either the 'short' or 'long' EMP preparations. The samples were then scraped onto the surface of the skin. Administration was achieved by massaging the solution for 30 s using small circular motions within a 3 cm² circular area. To maintain consistency between administrations, a small hand-held applicator was designed resulting in an application force of 0.25 N (as described in AU2012905650). After administration, the payload was left on the skin for 5 min followed by removal of excess solution with cotton swabs. The centre of the application area was excised using a 6 mm circular punch biopsy. A total of 6 samples were done per group. Each sample was stored at -20°C prior to analysis. Figure 12a represents a dermoscopy image of the pig skin with topical application of sodium fluorescein only (no EMP). Figures 12b and 12c illustrate dermoscopy images of sodium fluoroscein delivered by short EMPs (Figure 12b) delivery and long EMPs (Figure 12c). The white rod-like structures in Figures 12a to c are hairs. Figures 12d represents fluorescence confocal microscopy mosaic images of the pig skin with topical application of sodium fluorescein only (no EMP). Figures 12e and 12f illustrate dermoscopy images of sodium fluoroscein delivered by short EMPs (Figure 12e) and long EMPs (Figure 12f). Figures 12g to 12i are magnified images of the square insets shown in Figures 12d, 12e and 12f respectively. Figures 12 j to l illustrate the cross-sectional sodium fluorescein intensity within the magnified images shown in Figures 12g to 12i respectively. The images in Figure 12d to 12l have been pseudo-colored in greyscale, where black corresponds to 0 (min) fluorescence intensity and white corresponds to 255 (max) fluorescence intensity, as shown on the scale superimposed on Figure 12l.

Assessment of the dermoscopy images showed no visible damage to the skin (Figures 12b to 12c) post treatment with either the 'short' or 'long' EMPs. Topical application of sodium fluorescein without EMPs was used for comparison as illustrated in Figure 12a. Topical application (no EMP) of sodium flourescein resulted in a relatively uniform delivery profile across the treatment area. Higher levels of intensity were detected within and around the hair follicles. However, minimal fluorescence was detected below the surface of the skin as illustrated in Figures 12g and 12j. A similar delivery profile was detected for the 'short' EMPs. Additionally, pockets of high levels of intensity were detected, which corresponded to an increased depth of detectable signal within the skin as illustrated in Figures 12h and 12k.

Enhanced sodium fluorescein penetration using the 'long' EMPs resulted in greater fluorescein signal.

The 'long' EMPs showed a relatively uniform distribution of sodium fluorescein as illustrated in Figure 12f. However, much greater delivery was achieved with high signal within the stratum corneum and viable epidermis, as well as delivery into the upper dermis as illustrated in Figures 12i and 12l.

Following successful EMP administration and enhanced delivery to pig skin *ex vivo,* EMPs were applied to the flank of live pigs *in vivo.* Negligible damage was observed in the treatment areas post EMP administration with minimal erythema. Following application the topical alone (no EMP) and 'short' EMP treatment areas looked similar. The 'long' EMPs resulted in greater fluorescein signal.

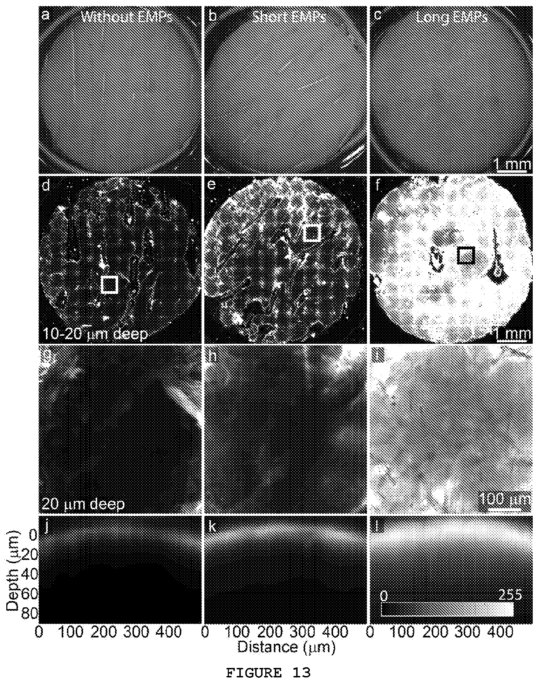

Figure 13 represents comparison of delivery of sodium fluorescein to *in vivo* pig skin. *In vivo* experiments were conducted on 6 female Landrace weaner pigs weighing approx. 10-15 kg (Centre for Advanced Animal Science, The University of Queensland Gatton Campus, Australia). The pigs were anaesthetized intramuscularly by co-administration of Butorphanol (0.15 mg/kg) and Zoletil (5 mg/kg) followed by intubation with isoflurane to maintain unconsciousness. Following anesthesia the flanks of the pigs were washed with soapy water and patted dry. The flank was then shaved gently using an electric razor. The flank was marked, to separate the application areas (2 cm diameter circles, 2 cm apart). Payload administration and amounts were done using the same parameters as for ex vivo. After all the groups / samples were applied and swabbed, the pig was turned over and the procedure repeated to the other flank (resulting in 12 samples per group). Finally, the pig was culled and the center of the application areas was excised using a 6 mm circular punch biopsy. Each sample was placed on dry-ice followed by storage at -20°C prior to analysis.

Figure 13a represents dermoscopy image of the pig skin with topical application of sodium fluorescein only. Figures 13b and 13c illustrate dermoscopy images of sodium fluorescein delivered by short EMPs (Figure 13b) delivery and long EMPs as illustrated in Figure 13c. Again, the white rod-like structures in Figures 13a to c are hairs. Figures 13d to 13f represent fluorescence confocal microscopy mosaic images of the pig skin with topical (no EMP), short EMP and long EMP application of sodium fluorescein. Figures 13g to 13i are magnified images of the square insets illustrated in Figures 13d, 13e and 13f respectively. Figures 13 j to l illustrate the cross-sectional sodium fluorescein intensity within the magnified images shown in Figures 13g to 13i respectively. The images in Figure 13d to 13l have been pseudo-colored in greyscale, where black corresponds to 0 (min) fluorescence intensity and white corresponds to 255 (max) fluorescence intensity, as shown on the scale superimposed on Figure 13l.

Assessment of the dermoscopy images in Figures 13a to 13c showed no visible damage to the skin. The topically applied sodium fluorescein alone resulted in a relatively uniform delivery profile across the treatment area, with higher levels of intensity around the hair follicles as illustrated in Figure 13d. Minimal fluorescence was detected below the surface of the skin as shown in Figure 13g and 13j. The 'short' EMP enhanced delivery as illustrated in Figure 13e resulted in greater signal than what was observed for the topical application of sodium fluorescein alone (no EMP) and corresponding *ex vivo* samples. Although brighter, signal was still localized to the upper layers of the skin as illustrated in Figure 13h and 13k.

The 'long' EMPs resulted in a dramatically increased amount of signal detected as illustrated in Figure 13f. The 'long' EMPs showed a relatively uniform distribution of sodium fluorescein. Much greater delivery of sodium fluorescein was achieved with high signal detected within the stratum corneum and viable epidermis, as well as delivery into the upper dermis as illustrated in Figure 13i and 13l.

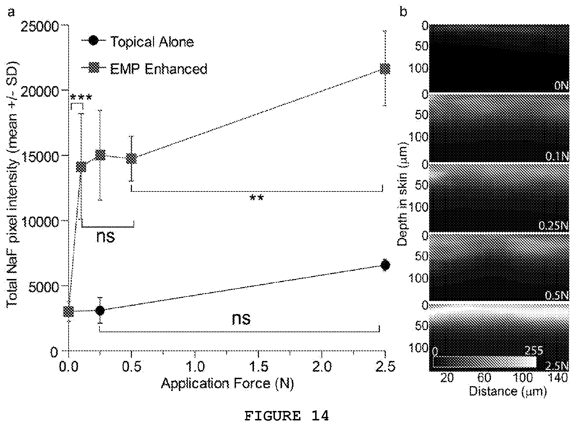

Figure 14 illustrates the effect of application force on EMPs for delivery of sodium fluorescein. Mixtures were formed by taking 50µl of sodium fluorescein dye (1mg/mL), with or without 5mg of silica EMP. Figure 14a illustrates a graphical comparison of variation in pixel intensity of sodium fluorescein over varying application force on long EMPs between topically applied sodium fluorescein (without EMP) and low angle application of EMP (volar forearm of four healthy human volunteers, significance p < 0.05 based on a one-way ANOVA, Tukey's multiple comparison test). Figure 14b shows fluorescence microscopy images of a treatment area with varying levels of application force applied on the EMPs using the applicator as described in co-pending provisional patent application AU2012905650. The images in Figure 14b have been pseudo coloured in greyscale, where black corresponds to 0 (min) fluorescence intensity and white corresponds to 255 (max) fluorescence intensity, as shown on the superimposed scale. Figure 14 illustrates that minimum force is required for successful EMP delivery. These data show that this system works under a variety of application forces and that delivery can be tuned by varying application force. Further, even though the delivery amount changes, the delivery profile for field-directed delivery remains relatively constant. This conveniently enables the delivery of a specific amount of drug across a defined field using the same EMP formulation, e.g. treating photoaged skin on the face may require a lower dosage than the forearm and in this scenario the final formulation would remain the same and the application force would just be less for the face. Such performance is a unique attribute of delivery across epidermal layers using EMPs.

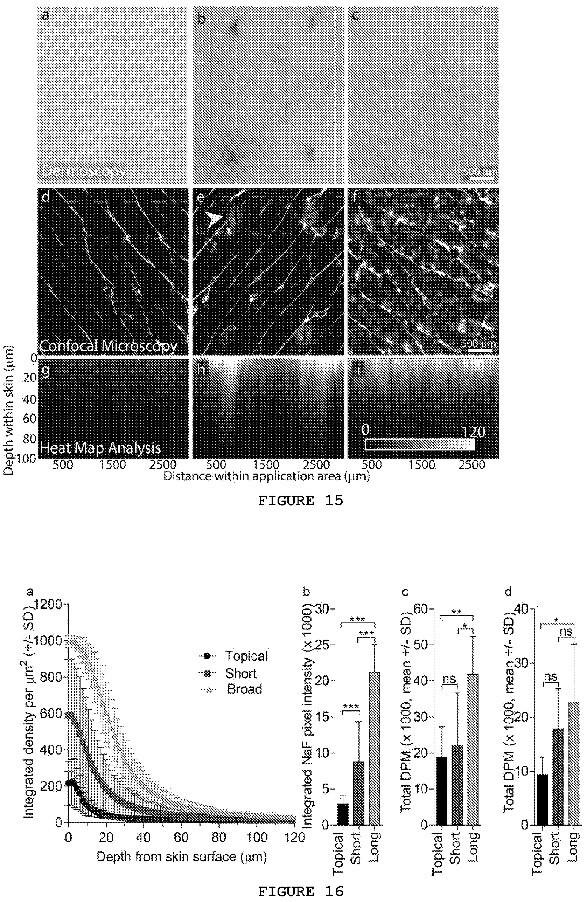

Figure 15 illustrates a comparison of the delivery profile of sodium fluorescein obtained by topical application alone (no EMP), by using fixed substrate microneedles and by using long EMPs respectively.

In the case of delivery using fixed substrate microneedles, a 2 x 3 array of microneedles, 750 µm in length were laser-cut from 50 µm stainless steel. The microneedles were applied onto the volar forearm of human volunteers using an applicator. The microneedles were removed followed by application of 50 µL of 0.01 mg/mL sodium fluorescein solution. The sodium fluorescein was gently massaged into the microneedle tracks for 30 s with 0.25 N of force.

In the case of delivery using long EMPs, 50 µL of the sodium fluorescein solution was gently mixed with 5 mg of EMP. The mixture was placed on the surface of the skin and gently massaged with the applicator as described in co-pending provisional patent application AU2012905650 using the above technique.

In the case of topical application alone (no EMP), 50 µL of the sodium fluorescein solution was pipetted onto the surface of the skin and gently massaged using the same conditions as for using long EMPs. For all groups, excess sodium fluorescein was wiped off followed by rinsing and drying of the skin prior to imaging by fluorescent confocal microscopy.

Figures 15a to 15c are dermoscopy images of skin with three types of samples. Figure 15a represents dermoscopy image of skin with topical application of sodium fluorescein only (no EMP). Figures 15b and 15c illustrate dermoscopy images of sodium fluoroscein delivered by fixed substrate microneedles (Figure 15b) and by EMPs (Figure 15c), respectively. Figures 15d to 15f are confocal microscopic images of skin with three types of samples. Figure 15d represents confocal microscopic image of skin with topical application (no EMP) of sodium fluorescein only. Figures 15e and 15f illustrate confocal microscopy images of sodium fluorescein delivered by fixed substrate microneedles (Figure 15e) and by EMPs (Figure 15f) respectively. Figures 15 g to 15i are images obtained by heat map analysis of skin with three types of samples. The images in Figure 15 have been pseudo coloured in greyscale, where black corresponds to 0 (min) fluorescence intensity and white corresponds to 120 (max) fluorescence intensity, as shown on the superimposed scale superimposed. Figure 15g represents a heat map analysis of skin with topical application of sodium fluorescein only (without EMP). Figures 15h and 15i illustrate heat map analysis of skin samples in which sodium fluorescein is delivered by fixed substrate microneedles (Figure 15h) and by EMPs (Figure 15i) respectively. The representative cross-sectional heat-maps clearly demonstrate that fixed substrate microneedle delivery results in focal delivery of sodium fluorescein perpendicular to the surface of the skin as shown in Figure 15h. However, angled penetration from the EMPs maximizes skin disruption resulting in a continuous 'field- directed' delivery profile as shown in Figure 15i.

After sodium fluorescein administration, the volunteer's skin was examined to determine the presence and degree of inflammation ('erythema') arising from topical alone (NaF), microneedle (MN) and elongate microparticle (EMP) delivery, respectively. MNs and EMPs resulted in very mild inflammation post administration increasing within the first two hours. After 24 hours, inflammation had decreased with minimal inflammation seen for the EMPs after 48 hrs. However, MN tracks were still visible even after 48 hours, with inflammation directly around the MN sites.

Accordingly, administration using EMPs results in a relatively uniform and continuous delivery profile in the EMP treated area within upper layers of the skin, with lower inflammation as compared with administration using MNs.

It is important to appreciate that Example 2 demonstrates that an *angle* at which microparticles are applied to the skin determines the amount of skin penetration *i.e.* a more acute angle to the skin results in less penetration than a more obtuse angle. Therefore, a low angle of EMP penetration from the surface of the skin maximizes the volume of EMP within the epidermis and therefore a potential increase in drug delivery. Another way of further optimizing the EMPs may be to produce curved microparticles that are 'bent' to the optimal angle.

An added benefit to having a low angle of penetration is that by using a low angle of penetration, EMPs are able to disrupt the skin, increasing permeability while minimizing damage to the pain receptors and capillaries within the dermis. Minimal erythema was observed by administration of EMPs. This is unlike fixed substrate microneedle delivery, where the projections penetrate into the epidermis and dermis that results in visible puncture sites.

### Example 3

### Delivery of small molecules and vaccines.

Example 3 illustrates enhanced delivery of sodium fluorescein, Vitamins A, B₃ and E and the photodynamic therapy drug aminolevulinic acid by using EMPs of the disclosure. It also demonstrates an enhanced immune response to a DNA vaccine when the vaccine is delivered using EMP compared to intramuscular delivery.

Referring to Figure 16, the quantification and delivery profile of sodium fluorescein, Vitamins A (³H-all-*trans* retinoic acid), and B₃ (³H-nicotinamide) are shown when delivered using topical application (No EMP), short EMP and long EMP. Vitamin B3 (³H-nicotinamide) has been recently shown to prevent non-melanoma skin cancers and is a potential treatment for reversing skin photoageing. Excised pig ears sourced from a local abattoir were washed with soap and water followed by rinsing. The ears were stored at -20°C until required. The ears were thawed and then gently shaved to remove excessive hair, then rinsed and pat dried. The ventral side of the ear was then separated from the cartilage using a scalpel. The excised skin was pinned down on a cork-board maintaining slight tension across the surface. For EMP delivery, 50 µL of the payload solution (1 mg/mL sodium fluorescein, 0.01 mCi/mL vitamin A or 0.01 mCi/mL, vitamin B3 respectively) was gently mixed with 5 mg of either the 'short' or 'long' EMP preparations. The samples were then scraped onto the surface of the skin.

Administration was achieved by massaging the payload mixture for 30 s using small circular motions within a 3 cm² circular area. To maintain consistency between administrations, a small hand-held applicator (as described in AU2012905650) was used having an application force of 0.25 N. The EMP groups were compared to samples without EMPs administered to the skin using the same conditions described above. After administration, the payload was left on the skin for 5 min followed by removal of excess solution with cotton swabs. The center of the application area was excised using a 6 mm circular punch biopsy. A total of 6 samples were done per group. Each sample was stored at -20°C prior to analysis.

The superior ability of 'long' EMP to enhance the penetration of sodium fluorescein as demonstrated in Fig. 5 for thawed human skin was also demonstrated in Fig 16a in freshly excised *ex vivo* pig skin. This pig skin model was used to show the same enhancement of delivery for compounds other than sodium fluorescein. The delivery profile of sodium fluorescein integrated density within the skin is illustrated in Figure 16b. The integrated density of sodium fluorescein is directly related to the concentration of the drug within the skin. Therefore, higher levels of integrated density are directly related to higher levels of drug penetration. Figures 16c and 16d show ³H-vitamin B₃ delivery and ³H-vitamin A delivery to pig skin, respectively. In this case, skin was pinned out taut on a cork board, epidermal side uppermost. Tritiated compound was applied to the skin surface and left for 15 minutes. Radiolabeled treated samples were placed in separate scintillation vials with the addition of 1 ml of tissue solubiliser. The samples were solubilised overnight at 55°C overnight. All samples were then vortexed thoroughly prior to the addition of 10 mL of scintillation fluid and vortexed again. Samples were then counted using a beta counter (PerkinElmer TriCarb Model 2810TR). The level of significance was accepted at p < 0.05 based on a one-way ANOVA, Tukey's multiple comparison test. (NaF = sodium fluorescein, DPM = disintegrations per minute). In similar fashion to NaF, these data demonstrate that vitamin B₃ delivery is significantly enhanced by long EMP compared to short EMP.

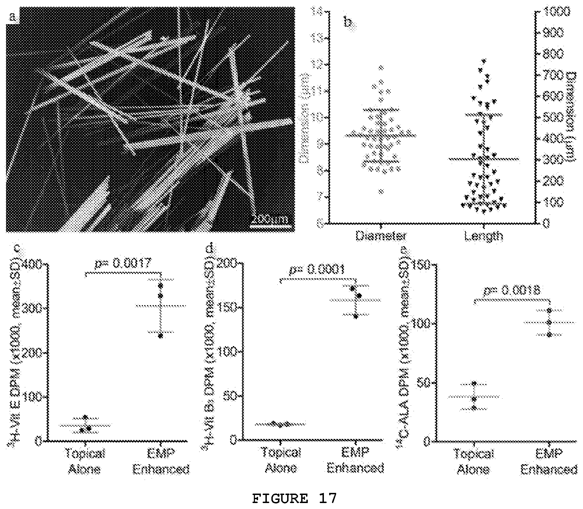

Figure 17 shows the characterization and *ex vivo* delivery of ³H-vitamin E, ³H-vitamin B₃ and ¹⁴C-aminolevulinic acid to human skin using EMPs of different lengths. Vitamin E is a common topical antioxidant used to treat and prevent skin photodamage. ¹⁴C-aminolevulinic acid is a topical photodynamic therapy drug with poor skin penetration profile.

Excised skin obtained previously from female, Caucasian, abdominal plastic surgery patients was thawed then rinsed and pat dried. The skin was pinned down on a cork-board maintaining slight tension across the surface. For delivery using EMP's of the present disclosure, 50 µL of the payload solution (³H-all-*trans* retinoic acid, 0.01 mCi/mL ³H-nicotinamide or 0.01 mCi/mL ¹⁴C-aminolevunic acid respectively) was gently mixed with 5 mg of EMPs. The samples were then scraped onto the surface of the skin. Administration was achieved by massaging the payload mixture for 30 s using small circular motions within a 3 cm² circular area. To maintain consistency between administrations, a small hand-held applicator was used at an application force of 0.25 N (as described in AU2012905650). The EMP groups were compared to samples without EMP administered to the skin using the same conditions described above. After administration, the payload was left on the skin for 5 min followed by removal of excess solution with cotton swabs. The center of the application area was excised using a 6 mm circular punch biopsy. A total of 3 samples were done per group. Each sample was stored at -20°C prior to analysis. Figure 17a shows scanning electron microscopy images of the EMPs used in the experiments. Figure 17b shows characterization of the elongate microparticle diameter and length. Figures 17c to 17e show the delivery of ³ H vitamin E (³ H-Vit E), ³ H vitamin B₃ (³ H-Vit B3) and ¹⁴C aminolevulinic acid (¹⁴C-ALA) to the skin, respectively. The level of significance was accepted at p < 0.05 based on an unpaired student t-test. (DPM = Disintegrations per minute). These data confirm that EMP can be successfully used to enhance the skin penetration of structurally unrelated compounds.

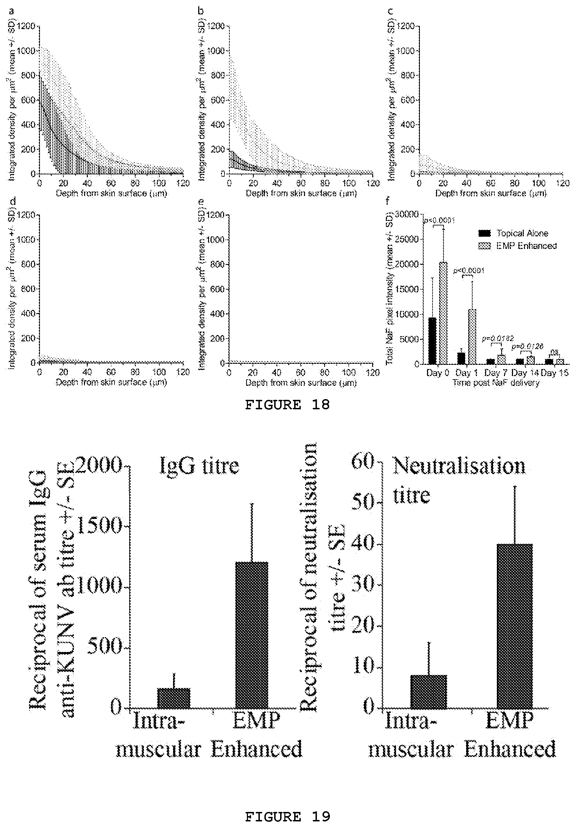

Referring to Figure 18 quantification of sodium fluorescein using *in vivo* fluorescence confocal microscopy in four volunteers over two weeks was discovered and is illustrated. Mixtures were formed by taking 50µl of sodium fluorescein dye (1mg/mL), with or without 5mg of silica EMP. Figures 18a to 18e illustrate the delivery profile of sodium fluorescein integrated density within the skin using a force of 2.5N i.e. 18a: 0weeks, 18b: 24-hours, 18c: 7 days, 18d: 14-days, 18e: 15 days following dye application when delivered with or without 'long' EMP. A uniform force was applied using a plastic applicator as described in AU2012905650 which allowed a consistent 2.5N force to be applied to the skin surface and helped orientate EMP at an acute angle for better skin penetration . 'Integrated density' was calculated and refers to the integrated density of sodium fluorescein pixel intensity. Topically applied sodium fluorescein without EMP resulted in a significantly decreased fluorescence signal after 24 hours leveling to background signal within a week. However, by comparison after two weeks, a slight signal was still detected for EMP enhanced delivery with no signal detected by day 15 following NaF administration. Figure 18f refers to the sum of the total sodium fluorescein integrated density within the skin. The level of significance was accepted at p < 0.05 based on a one-way ANOVA, Tukey's multiple comparison test. These data demonstrate that topical application of sodium fluorescein with 'long' EMP provides a more lasting and deeper penetration.

Referring to Figure 19, the superior ability of EMP to induce an immune response to a non-infectious DNA-based West Nile virus (WNV) vaccine compared to intramuscular delivery was discovered as illustrated. Figure 19 shows the immune response in mice treated with vaccine by either intramuscular inoculation or transdermal application with long EMP. This vaccine has been described by others (Hall et al, 2003, PNAS 100(18):10460-10464). It utilises the full length genome of the Kunjin strain (pKUN1) and induces a potent immune response by mimicking viral infection. Mice were either inoculated with pKUN1 by the intramuscular route or received a transdermal dose of long EMP mixed with pKUN1. An enhanced immunoglobulin response (IgG titre) was observed when the vaccine was delivered by EMP compared to the conventional intramuscular route. In Figure 19 'neutralisation titre' refers to the amount of virus specific antibody that must be administered to a mouse to block establishment of the infection. The amount of antibody required to block establishment of infection in the case of mice inoculated with pKUN1 by EMP was much greater than when it was delivered intramuscularly. These data suggest that vaccine delivery by EMP produces a more significant infection than via the intramuscular route. This implies that presenting vaccine to skin antigen presenting cells (APC) using EMP produces a more significant immune response than intramuscular delivery.

Examples 4 and 5 relate to tests conducted on EMPs fabricated from silicon, which are not part of the invention. The trends observed in the test results are expected to be also applicable to silica EMPs given the similar mechanical properties of silica and silicon. In both Examples 4 and 5, elongate microparticles (EMPs) were fabricated from silicon using deep reactive ion etching. Lithography was used to pattern a silicon wafer that was then coated with SU-8 photoresist. After a post exposure bake, the resist was developed in polypropylene glycol monomethylethyl acetate. The SU8 structures remaining served as a physical mask during etching, which were etched using a Bosch process. The etched EMPs were washed three times in acetone, followed by three washes in 100% ethanol and lastly three times in distilled water. Prior to application the EMPs were removed from the silicon wafer.

### Example 4:

### Influence of EMP diameter on penetration and delivery

The ability of silicon EMPs of various diameters to penetrate the skin was determined by application of a mixture of sodium fluorescein and EMPs to the volar forearm of four human, healthy volunteers using a plastic applicator as described in provisional patent application AU2012905650. 50 µL of the sodium fluorescein solution having a concentration of 1 mg/mL was mixed with 5mg of silicon EMPs and applied to the forearm using a gentle massage motion with the applicator. A control containing no EMPs was also pipetted onto the surface of the skin and gently massaged using the same conditions as for EMP containing solutions. The volunteers all received four dye solutions to four different skin regions on the forearm on the same day. Three of the dye solutions contained EMPs of respective different diameters and the fourth dye solution contained no EMPs. For all groups, excess sodium fluorescein was wiped off followed by rinsing and drying of the skin prior to imaging by fluorescent confocal microscopy.

### Results

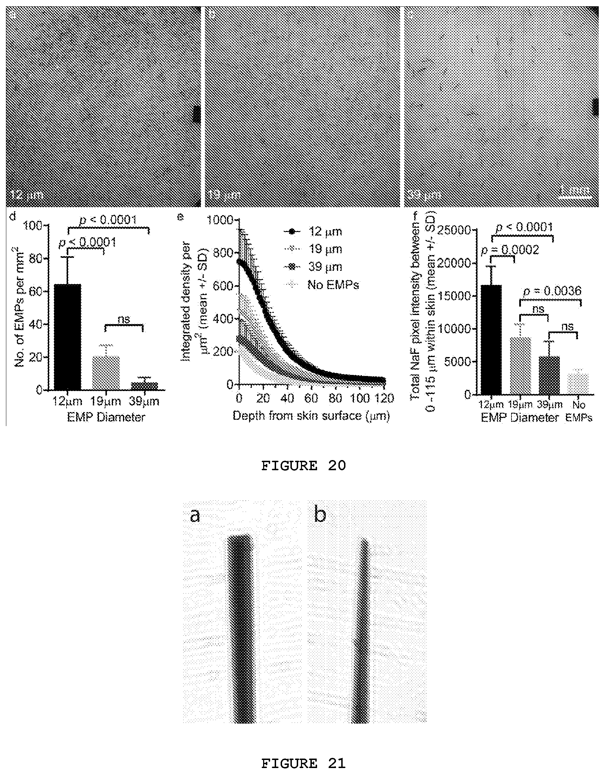

Three different populations of EMPs were used to assess the influence of EMP diameter on penetration and delivery. The respective diameters for each population were 11.7 ± 1.7 µm, 19.1 ± 2.4 µm and 38.6 ± 1.6 µm, (rounded off to 12, 19 and 39**µm** respectively) and their respective lengths were 242.2 ± 4.0 µm, 244.3 ± 30.1 µm and 329.2 ± 13.9 µm (Figure 20 a-c). Grouped data was analysed using a one-way ANOVA with a Tukey's multiple comparison test. The level of significance was accepted at p < 0.05.

The results are illustrated in Figure 20.

The variation in diameter resulted in a significant difference in the number of EMPs that penetrated the skin (Figure 20a-d). The smallest diameter (12 µm) resulted in 64 ± 17 EMPs per mm², followed by 20 ± 7 EMPs per mm² when using 19 µm diameter EMPs and only 4 ± 4 EMPs per mm² when using the largest diameter EMPs (39 µm). The reduction in EMPs per mm² corresponded to a decrease in the amount of delivered sodium fluorescein within the skin (Figure 20e-f). Under the administration conditions, the smallest diameter EMPs resulted in a significant increase in delivery over the 19 µm EMPs, 39 µm EMPs and without EMPs (*p* = 0.0002, *p* < 0.0001, *p* < 0.0001, respectively). The 19 µm EMPs resulted in no significant difference to the 39 µm EMPs but there was a significant difference without EMPs (*p* = 0.0036). Finally, there was no significant difference in the amount of sodium fluorescein detected between the largest diameter EMPs (39 µm) and topical application without EMPs.

### Example 5

### Influence of EMP end geometry on penetration

EMPs 11.7 ± 1.7 µm in diameter and 242.2 ± 4.0 µm in length were used to assess the effect end geometry has on penetration. Each EMP had two different types of end geometries One end geometry comprised a blunt tip end such that the end face of the EMP had an included angle of 180° (see Figure 21a). The second type of end geometry comprised a tapered tip end such that the end face had an included angle of 6° (Figure 21b). The results indicated that the tapered end geometry preferentially penetrated the skin resulting in 71 ± 6% of the EMPs penetrating in this manner and only 29 ± 6% of penetrations occurring via the blunt ends.

In the claims which follow, and in the preceding description, except where the context requires otherwise due to express language or necessary implication, the word "comprise" and variations such as "comprises" or "comprising" are used in an inclusive sense, i.e. to specify the presence of the stated features but not to preclude the presence or addition of further features in various embodiments of the apparatus and method as disclosed herein.

References to the background art herein do not constitute an admission that the art forms a part of the common general knowledge of a person of ordinary skill in the art. The references are also not intended to limit the application of the method and composition as disclosed herein.

## Claims

1. Non-therapeutic method for delivering a compound through a biological barrier, the method including:
a) providing a composition including 1) a plurality of elongate biocompatible microparticles and 2) the compound; and
b) applying a force of between 0.01 Newtons and 10 Newtons by rubbing or massaging the composition on a surface of the barrier manually or by using an applicator, so that at least some of the microparticles penetrate the biological barrier and thereby facilitate delivery of the compound through the biological barrier;
wherein the microparticles:
i) have a geometry and strength sufficient to penetrate the biological barrier;
ii) comprise silica;
iii) have an average length of from 90 µm to 510 µm; and
iv) have a width or diameter of 20 µm or less.

2. The method of claim 1, wherein the compound is a bioactive compound.

3. The method of claim 1 or claim 2, wherein the biological barrier is a stratum corneum of skin or mucosa.

4. The method of any one of the preceding claims, wherein the force is between 0.1 and 10 Newtons.

5. The method of any one of the preceding claims, wherein the microparticles penetrate the biological barrier at an acute angle with respect to the biological barrier, such as at an angle of less than 45 degrees, such as between approximately 5 to 30 degrees, for example between 7 and 25 degrees.

6. A composition for use in delivering a bioactive compound through a biological barrier, including:
(1) a plurality of elongate biocompatible microparticles, wherein the microparticles:
i) have a geometry and strength sufficient to penetrate the biological barrier upon application of a force of between 0.01 Newtons and 10 Newtons by rubbing or massaging manually or by using an applicator;
ii) comprise silica;
iii) have an average length of from 90 µm to 510 µm; and
iv) have a width or diameter of 20 µm or less; and
(2) the bioactive compound to be delivered;
wherein:
(a) the bioactive compound forms a coating on the elongate biocompatible silica microparticles; or
(b) the composition comprises a gel, emulsion, cream, ointment or solution.

7. The composition of claim 6, wherein the biological barrier is a stratum corneum of skin or mucosa.

8. The composition of claim 6 or claim 7, wherein the microparticles penetrate the biological barrier at an acute angle with respect to the biological barrier, such as at an angle of less than 45 degrees, such as between approximately 5 to 30 degrees, for example between 7 and 25 degrees.

9. The composition of any one of claims 6 to 8, wherein the width of the microparticles is less than 12 µm.

10. The composition of any one of claims 6 to 9, wherein at least some of the microparticles have one or more substantially flat ends and/or one or more substantially convex ends.

11. The composition of any one of claims 6 to 10, wherein the strength of the microparticles is such as to withstand applied forces of at least 0.01 Newtons.

12. The composition of any one of claims 6 to 11, wherein the bioactive compound is selected from a therapeutic compound, a cosmeceutical, a pharmaceutical, a nutraceutical, a diagnostic agent and a vaccine.

13. The composition of any one of claims 8 to 12, wherein at least some of the microparticles are configured to at least withstand pressure of up to 13MPa.

14. The composition of any one of claims 6 to 13 wherein at least some of the microparticles have a tapered end having an included angle of less than 20 degrees, such as less than 10 degrees.

## Patentansprüche

1. Nicht-therapeutisches Verfahren zur Verabreichung einer Verbindung durch eine biologische Barriere, wobei das Verfahren umfasst:
a) Bereitstellen einer Zusammensetzung, umfassend 1) eine Vielzahl von länglichen biokompatiblen Mikropartikeln und 2) die Verbindung; und
b) Aufbringen einer Kraft zwischen 0,01 Newton und 10 Newton durch Reiben oder Massieren der Zusammensetzung auf einer Oberfläche der Barriere von Hand oder unter Verwendung eines Applikators, so dass zumindest einige der Mikropartikel die biologische Barriere durchdringen und dadurch die Abgabe der Verbindung durch die biologische Barriere erleichtern;
wobei die Mikropartikel:
i) eine Geometrie und Festigkeit aufweisen, die ausreicht, um die biologische Barriere zu durchdringen;
ii) Silika umfassen;
iii) eine durchschnittliche Länge von 90 µm bis 510 µm aufweisen; und
iv) eine Breite oder einen Durchmesser von 20 µm oder weniger haben.

2. Verfahren nach Anspruch 1, wobei die Verbindung eine bioaktive Verbindung ist.

3. Verfahren nach Anspruch 1 oder Anspruch 2, wobei die biologische Barriere ein Stratum corneum der Haut oder Schleimhaut ist.

4. Verfahren nach einem der vorhergehenden Ansprüche, wobei die Kraft zwischen 0,1 und 10 Newton liegt.

5. Verfahren nach einem der vorhergehenden Ansprüche, wobei die Mikropartikel die biologische Barriere in einem spitzen Winkel in Bezug auf die biologische Barriere durchdringen, beispielsweise in einem Winkel von weniger als 45 Grad, beispielsweise zwischen etwa 5 und 30 Grad, zum Beispiel zwischen 7 und 25 Grad.

6. Zusammensetzung zur Verwendung bei der Abgabe einer bioaktiven Verbindung durch eine biologische Barriere, umfassend:
(1) eine Vielzahl von länglichen biokompatiblen Mikropartikeln, wobei die Mikropartikel:
i) eine Geometrie und Festigkeit aufweisen, die ausreicht, um die biologische Barriere bei Anwendung einer Kraft zwischen 0,01 Newton und 10 Newton durch Reiben oder Massieren von Hand oder mit einem Applikator zu durchdringen;
ii) Silika umfassen;
iii) eine durchschnittliche Länge von 90 µm bis 510 µm haben; und
iv) eine Breite oder einen Durchmesser von 20 µm oder weniger haben; und
(2) die zu verabreichende bioaktive Substanz;
wobei:
(a) die bioaktive Verbindung eine Beschichtung auf den länglichen biokompatiblen Silika-Mikropartikeln bildet; oder
(b) die Zusammensetzung ein Gel, eine Emulsion, eine Creme, eine Salbe oder eine Lösung umfasst.

7. Zusammensetzung nach Anspruch 6, wobei die biologische Barriere ein Stratum corneum der Haut oder Schleimhaut ist.

8. Zusammensetzung nach Anspruch 6 oder 7, wobei die Mikropartikel die biologische Barriere in einem spitzen Winkel in Bezug auf die biologische Barriere durchdringen, beispielsweise in einem Winkel von weniger als 45 Grad, beispielsweise zwischen etwa 5 und 30 Grad, zum Beispiel zwischen 7 und 25 Grad.

9. Zusammensetzung nach einem der Ansprüche 6 bis 8, wobei die Breite der Mikropartikel weniger als 12 µm beträgt.

10. Zusammensetzung nach einem der Ansprüche 6 bis 9, wobei mindestens einige der Mikropartikel ein oder mehrere im Wesentlichen flache Enden und/oder ein oder mehrere im Wesentlichen konvexe Enden aufweisen.

11. Zusammensetzung nach einem der Ansprüche 6 bis 10, wobei die Festigkeit der Mikropartikel so beschaffen ist, dass sie angewandten Kräften von mindestens 0,01 Newton standhalten.

12. Zusammensetzung nach einem der Ansprüche 6 bis 11, wobei die bioaktive Verbindung ausgewählt ist aus einer therapeutischen Verbindung, einem kosmetischen Mittel, einem Arzneimittel, einem Nahrungsergänzungsmittel, einem diagnostischen Mittel und einem Impfstoff.

13. Zusammensetzung nach einem der Ansprüche 8 bis 12, wobei mindestens einige der Mikropartikel so konfiguriert sind, dass sie mindestens einem Druck von bis zu 13 MPa standhalten.

14. Zusammensetzung nach einem der Ansprüche 6 bis 13, wobei mindestens einige der Mikropartikel ein verjüngtes Ende mit einem eingeschlossenen Winkel von weniger als 20 Grad, beispielsweise weniger als 10 Grad, aufweisen.

## Revendications

1. Procédé non thérapeutique pour administrer un composé à travers une barrière biologique, le procédé incluant les étapes consistant à :
a) fournir une composition incluant 1) une pluralité de microparticules biocompatibles allongées et 2) le composé ; et
b) appliquer une force comprise entre 0,01 Newton et 10 Newtons en frottant ou en massant la composition sur une surface de la barrière manuellement ou en utilisant un applicateur, de sorte qu'au moins certaines des microparticules pénètrent dans la barrière biologique et facilitent ainsi l'administration du composé à travers la barrière biologique;
dans lequel les microparticules:
i) présentent une géométrie et une résistance suffisantes pour pénétrer la barrière biologique;
ii) comprennent de la silice;
iii) présentent une longueur moyenne de 90 µm à 510 µm; et
iv) présentent une largeur ou un diamètre de 20 µm ou moins.

2. Procédé selon la revendication 1, dans lequel le composé est un composé bioactif.

3. Procédé selon la revendication 1 ou la revendication 2, dans lequel la barrière biologique est une couche cornée de la peau ou des muqueuses.

4. Procédé selon l'une quelconque des revendications précédentes, dans lequel la force est comprise entre 0,1 et 10 Newtons.

5. Procédé selon l'une quelconque des revendications précédentes, dans lequel les microparticules pénètrent dans la barrière biologique à un angle aigu par rapport à la barrière biologique, tel qu'à un angle inférieur à 45 degrés, tel qu'entre environ 5 et 30 degrés, par exemple entre 7 et 25 degrés.

6. Composition à utiliser pour administrer un composé bioactif à travers une barrière biologique, comprenant:
(1) une pluralité de microparticules biocompatibles allongées, dans laquelle les microparticules:
i) présentent une géométrie et une résistance suffisantes pour pénétrer la barrière biologique lors de l'application d'une force comprise entre 0,01 Newton et 10 Newtons en frottant ou en massant manuellement ou en utilisant un applicateur ;
ii) comprennent de la silice;
iii) présentent une longueur moyenne de 90 µm à 510 µm; et
iv) présentent une largeur ou un diamètre de 20 µm ou moins; et
(2) le composé bioactif à administrer;
dans laquelle:
(a) le composé bioactif forme un revêtement sur les microparticules de silice biocompatibles allongées; ou
(b) la composition comprend un gel, une émulsion, une crème, une pommade ou une solution.

7. Composition selon la revendication 6, dans laquelle la barrière biologique est une couche cornée de la peau ou des muqueuses.

8. Composition selon la revendication 6 ou la revendication 7, dans laquelle les microparticules pénètrent dans la barrière biologique selon un angle aigu par rapport à la barrière biologique, tel qu'un angle inférieur à 45 degrés, tel qu'entre environ 5 et 30 degrés, par exemple entre 7 et 25 degrés.

9. Composition selon l'une quelconque des revendications 6 à 8, dans laquelle la largeur des microparticules est inférieure à 12 µm.

10. Composition selon l'une quelconque des revendications 6 à 9, dans laquelle au moins certaines des microparticules présentent une ou plusieurs extrémités sensiblement plates et/ou une ou plusieurs extrémités sensiblement convexes.

11. Composition selon l'une quelconque des revendications 6 à 10, dans laquelle la résistance des microparticules est telle qu'elle résiste à des forces appliquées d'au moins 0,01 Newton.

12. Composition selon l'une quelconque des revendications 6 à 11, dans laquelle le composé bioactif est choisi parmi un composé thérapeutique, un produit cosmétique, un produit pharmaceutique, un produit nutraceutique, un agent diagnostique et un vaccin.

13. Composition selon l'une quelconque des revendications 8 à 12, dans laquelle au moins certaines des microparticules sont configurées pour au moins résister à une pression allant jusqu'à 13 MPa.

14. Composition selon l'une quelconque des revendications 6 à 13, dans laquelle au moins certaines des microparticules présentent une extrémité effilée présentant un angle inclus inférieur à 20 degrés, par exemple inférieur à 10 degrés.
